(19) Europäisches Patentamt
European Patent Office
Office européen des brevets

(11) **EP 4 699 560 A1**

(12) **EUROPEAN PATENT APPLICATION**

(43) Date of publication:
**25.02.2026 Bulletin 2026/09**

(21) Application number: **25190141.9**

(22) Date of filing: **17.07.2025**

(51) International Patent Classification (IPC):
**A61B 34/10** (2016.01)     **G06N 3/08** (2023.01)
**G06T 7/10** (2017.01)     **A61B 34/30** (2016.01)

(52) Cooperative Patent Classification (CPC):
**A61B 34/10; A61B 34/30; G06N 3/08; G06N 20/00;**
A61B 34/25; A61B 2034/107

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **20.08.2024 US 202463684875 P
30.10.2024 TW 113141733**

(71) Applicant: **National Taiwan University
Taipei 10617 (TW)**

(72) Inventors:
• **YEN, Ping-Lang
10617 Taipei (TW)**
• **CHUNG, Cheng-Yen
10617 Taipei (TW)**
• **ZUO, Hao-Cheng
10617 Taipei (TW)**

(74) Representative: **Winter, Brandl - Partnerschaft
mbB
Alois-Steinecker-Straße 22
85354 Freising (DE)**

(54) **ASSISTED SURGICAL PLANNING SYSTEM AND OPERATION METHOD THEREOF**

(57)     An assisted surgical planning system includes a host computer, a discriminative AI module, a user navigation interface, and a generative AI module. The discriminative AI module is electrically connected to the host computer, and is configured to form plural organ segmentation images based on plural CT images. The user navigation interface is electrically connected to the host computer, and is configured to display the organ segmentation images, an initial needle insertion path, plural suggested needle insertion paths, and a final needle insertion path. The generative AI module is electrically connected to the host computer, such that the host computer connects the user navigation interface to the generative AI module. The generative AI module is configured to generate the suggested needle insertion paths based on the organ segmentation images and the initial needle insertion path, and display the suggested needle insertion paths in the user navigation interface.

FIG. 1

**Description**

**BACKGROUND**

Technical field

The present disclosure relates to an assisted surgical planning system and an operation method of the assisted surgical planning system.

Description of Related Art

When preoperative needle insertion path planning for a puncture (e.g., a tumor puncture) is performed, doctors need to observe vital organs, hard-to-identify nerves, and small blood vessels, etc. on two-dimensional (2D) computed tomography (CT) images, and select appropriate image slices for path planning after repeated checks. The doctors' biggest concerns in path planning are risks of damaging blood vessels and causing complications, but such information is often not easily noticed in CT scans. The doctors may have to guess the locations of the vessels, or use a developer to take CT images, or use a contrast-enhanced computed tomography (CECT) method.

Finding access to safe needle insertion paths is challenging, highly dependent on the experience and expertise of the doctors, time-consuming and subject to subjective opinions. Additionally, since the doctors are not used to relying on three-dimensional (3D) image reconstruction when performing needle insertion path planning, they will mostly perform path planning in an axial view of a CT image. A slice plane of the axial view is easier for the doctors to imagine and more convenient for path planning. However, cross-plane needle insertion path planning is not easy for the doctors, and the doctors need to spend a lot of time before a surgery to adjust needle insertion paths and angles, and perform a CT scan after each stage of needle insertion. If it is found that the needle insertion path is not suitable or deviates from the original expectation during the surgery, it is necessary to make timely adjustment or even perform needle re-insertion to reduce the surgical risk.

**SUMMARY**

According to some embodiments of the present disclosure, an assisted surgical planning system comprises a host computer, a discriminative artificial intelligence (AI) module, a user navigation interface and a generative AI module. The discriminative artificial intelligence (AI) module is electrically connected to the host computer, and is configured to form plural organ segmentation images based on plural computed tomography (CT) images. The user navigation interface is electrically connected to the host computer, and is configured to display the organ segmentation images, an initial needle insertion path, plural suggested needle insertion paths, and a final needle insertion path. The generative AI module is electrically connected to the host computer, such that the host computer connects the user navigation interface to the generative AI module. The generative AI module is configured to generate the suggested needle insertion paths based on the organ segmentation images and the initial needle insertion path, and display the suggested needle insertion paths in the user navigation interface.

In some embodiments, the generative AI module is configured for selection and adjustment in the user navigation interface based on the suggested needle insertion paths to generate the final needle insertion path.

In some embodiments, the generative AI module comprises a user input unit configured to input an entry point, a target point, plural danger zones and a tumor into the organ segmentation images.

In some embodiments, the generative AI module further comprises a task unit configured to adjust the initial needle insertion path to generate the suggested needle insertion paths.

In some embodiments, the generative AI module further comprises a judgment unit configured to steer the suggested needle insertion paths away from the danger zones and adjust the target point to the center of the tumor.

In some embodiments, the generative AI module further comprises an output unit configured to generate coordinates and images of the suggested needle insertion paths.

In some embodiments, the assisted surgical planning system further comprises a visualization module. The visualization module is electrically connected to the generative AI module, and is configured to receive the CT images, the coordinates of the suggested needle insertion paths and coordinates of the final needle insertion path.

In some embodiments, the assisted surgical planning system further comprises a CT reslice module. The CT reslice module is electrically connected to the generative AI module, and is configured to receive the CT images and transmit the organ segmentation images to the generative AI module.

In some embodiments, the assisted surgical planning system further comprises a message module. The message module is electrically connected to the generative AI module, and is configured to transmit voice messages and type messages to the generative AI module.

In some embodiments, the assisted surgical planning system further comprises a robot module. The robot module is electrically connected to the generative AI module, and is configured to receive a command from the generative AI module to operate a robot having a puncture needle.

According to some embodiments of the present disclosure, an operation method of an assisted surgical planning system comprises: obtaining, by a discriminative AI module, plural CT images; setting an initial needle insertion path in a user navigation interface based on the CT images; forming, by the discriminative AI module, plural organ segmentation images based on the CT images; generating, by a generative AI module, plural suggested needle insertion paths based on the initial needle insertion path and the organ segmentation images, wherein the discriminative AI module, the generative AI module and the user navigation interface are electrically connected to a host computer; displaying the suggested needle insertion paths in the user navigation interface; and selecting and adjusting the suggested needle insertion paths from the user navigation interface, such that the generative AI module generates a final needle insertion path.

In some embodiments, the operation method of an assisted surgical planning system further comprises: selecting and adjusting the suggested needle insertion paths from the user navigation interface to form a modified path; and generating, by the generative AI module, plural other suggested needle insertion paths based on the modified path and the organ segmentation images.

In some embodiments, the operation method of an assisted surgical planning system further comprises: contextually training the generative AI module.

In some embodiments, contextually training the generative AI module comprises: inputting an entry point, a target point, plural danger zones and a tumor into the organ segmentation images, wherein the danger zones comprise arteries, veins, and blood vessels.

In some embodiments, contextually training the generative AI module further comprises: adjusting the initial needle insertion path to generate the suggested needle insertion paths; and generating coordinates and images of the suggested needle insertion paths.

In some embodiments, adjusting the initial needle insertion path to generate the suggested needle insertion paths comprises: steering the suggested needle insertion paths away from the danger zones; and adjusting the target point to the center of the tumor.

In some embodiments, the operation method of an assisted surgical planning system further comprises: receiving, by a visualization module electrically connected to the generative AI module, the CT images, the coordinates of the suggested needle insertion paths and coordinates of the final needle insertion path.

In some embodiments, the operation method of an assisted surgical planning system further comprises: receiving the CT images and transmitting the organ segmentation images to the generative AI module by a CT reslice module electrically connected to the generative AI module.

In some embodiments, the operation method of an assisted surgical planning system further comprises: transmitting, by a message module electrically connected to the generative AI module, voice messages and type messages to the generative AI module.

In some embodiments, the operation method of an assisted surgical planning system further comprises: receiving, by a robot module electrically connected to the generative AI module, a command from the generative AI module to operate a robot having a puncture needle.

In the above embodiments of the present disclosure, since the assisted surgical planning system includes the generative AI module and the host computer connects the user navigation interface to the generative AI module, the generative AI module can generate the suggested needle insertion paths based on the initial needle insertion path and the organ segmentation images, and the user navigation interface displays the suggested needle insertion paths for doctors' reference. The doctors can interact with the generative AI module through the user navigation interface, select and adjust the suggested needle insertion paths from the user navigation interface, such that the generative AI module generates the final needle insertion path. In this way, the assisted surgical planning system and the operation method thereof can effectively save the time of preoperative needle insertion path planning and reduce the influence of subjective opinions. In addition, the doctors can check, confirm, interact and adjust back and forth on customary two-dimensional (2D) images (e.g., organ segmentation images) when the assisted surgical planning system performs needle insertion path planning, so that the correctness of the needle insertion paths is improved, and the surgical risk can be further reduced.

**BRIEF DESCRIPTION OF THE DRAWINGS**

FIG. 1 illustrates a schematic diagram of an assisted surgical planning system according to one embodiment of the present disclosure;

FIG. 2 illustrates a sketch flow diagram of an operation method of the assisted surgical planning system according to one embodiment of the present disclosure;

FIG. 3 illustrates an image of a user navigation interface in FIG. 1 when a doctor performs initial path planning;

FIG. 4 illustrates an image of the user navigation interface in FIG. 1 when a final path is generated;

FIG. 5 illustrates a flow diagram of an operation method of an assisted surgical planning system according to one embodiment of the present disclosure;

FIG. 6 illustrates a block diagram of a generative AI module in FIG. 1;

FIG. 7 illustrates an image of the generative AI module in FIG. 6 when being contextually trained;

FIG. 8 illustrates images of the generative AI module when generating suggested needle insertion paths based on an initial needle insertion path and organ segmentation images according to one embodiment of the present disclosure;

FIG. 9 illustrates a schematic diagram of interaction between a doctor and the generative AI module according to one embodiment of the present disclosure;

FIG. 10 illustrates images when the initial needle insertion path is set according to one embodiment of the present disclosure;

FIG. 11 illustrates images when the organ segmentation images are formed according to one embodiment of the present disclosure;

FIG. 12 illustrates images when K-degree image reslicing is performed along the initial needle insertion path according to one embodiment of the present disclosure;

FIG. 13 illustrates images when the suggested needle insertion paths are generated according to one embodiment of the present disclosure;

FIG. 14 illustrates images when a final needle insertion path is generated according to one embodiment of the present disclosure;

FIG. 15 illustrates a flow diagram of an operation method of an assisted surgical planning system according to another embodiment of the present disclosure;

FIG. 16 illustrates a block diagram of the assisted surgical planning system according to one embodiment of the present disclosure; and

FIG. 17 illustrates a flow diagram of an operation method of an assisted surgical planning system according to yet another embodiment of the present disclosure.

## DETAILED DESCRIPTION

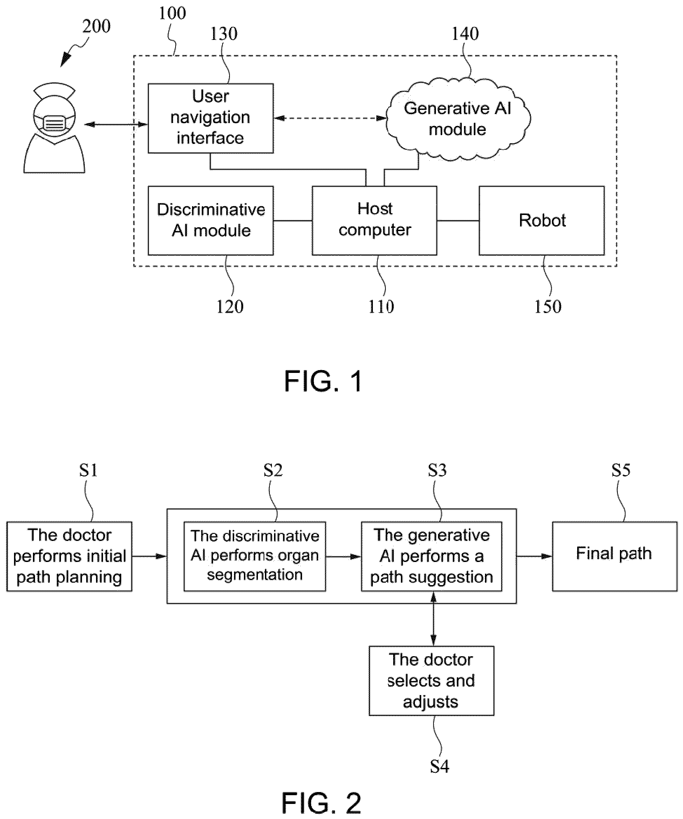

FIG. 1 illustrates a flow diagram of an assisted surgical planning system 100 according to one embodiment of the present disclosure. The assisted surgical planning system 100 includes a host computer 110, a discriminative AI module 120, a user navigation interface 130, a generative AI module 140, and a robot 150. The discriminative AI module 120 is electrically connected to the host computer 110. In some embodiments, the host computer 110 can be a Host computer. The user navigation interface 130 is electrically connected to the host computer 110, and may include a display screen. The generative AI module 140 is electrically connected to the host computer 110, such that the host computer 110 connects the user navigation interface 130 to the generative AI module 140. The robot 150 is electrically connected to the host computer 110 and can be a robotic arm having a puncture needle.

When in use, the discriminative AI module 120 can form plural organ segmentation images based on plural computed tomography (CT) images. The generative AI module 140 can generate plural suggested needle insertion paths based on the organ segmentation images and an initial needle insertion path provided by a user 200, and display the suggested needle insertion paths in the user navigation interface 130. The user 200 can be a doctor, e.g., a surgeon. The generative AI module 140 can perform selection and adjustment by the user 200 in the user navigation interface 130 based on the suggested needle insertion paths to generate a final needle insertion path through interaction. The user navigation interface 130 is configured to display the organ segmentation images, the initial needle insertion path, the suggested needle insertion paths, and the final needle insertion path. In some embodiments, the various needle insertion paths described above are, for example, needle insertion paths used in tumor punctures.

With the above configuration, the user 200 can observe the paths in the user navigation interface 130 and interact with the assisted surgical planning system 100. At the same time, the user navigation interface 130 is connected to the generative AI module 140 using the host computer 110. In this way, the user 200 can interact directly with the generative AI module 140 through the user navigation interface 130 and automatically visualize answers of the generative AI module 140 into the user navigation interface 130. After back-and-forth interaction and visualization, the assisted surgical planning system 100 generates a needle insertion path as the final insertion path which is transmitted to the robot 150 for subsequent path tracking.

Herein, "modules" and "units" can be implemented by software, hardware, or a combination of both. Plural different modules can be implemented in a same software or hardware structure, or one module can be implemented by plural different software or hardware structures. Hardware may include a processor, a memory, a hard disk, a sensor, or combinations thereof.

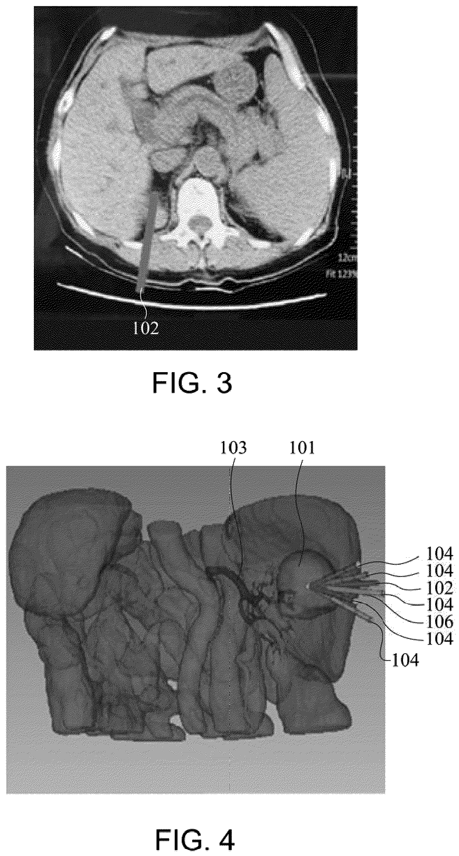

FIG. 2 illustrates a sketch flow diagram of an operation method of the assisted surgical planning system 100 according to one embodiment of the present disclosure. FIG. 3 illustrates an image of the user navigation interface in FIG. 1 when a doctor performs initial path planning. Referring also to FIGS. 2 and 3, first of all, in step S1, the doctor performs initial path planning. The doctor can perform initial path planning in CT images of a patient, for example, a brown path in FIG. 3 is an initial needle insertion path 102, and this step can provide a possible path range. Then, in step S2, the discriminative AI performs organ segmentation. Thus, the initial needle insertion path 102 and information about completion of the organ segmentation can be transmitted to the generative AI.

FIG. 4 illustrates an image of the user navigation interface 130 in FIG. 1 when the final path is generated. Reference is made also to FIG. 2 and FIG. 4, and then in step S3, the generative AI performs a path suggestion. In steps S2 and S3, the generative AI performing a cross-plane path suggestion can be implemented to generate light blue suggested needle insertion paths 104 as shown in FIG. 4. Then, in step S4, the suggested needle insertion paths 104 can be selected and adjusted by the doctor. In steps S3 and S4, the generative AI can interact with the doctor and adjust constantly. Then, in step S5, a needle insertion path is generated finally as a final path, e.g., a bright green final needle insertion path 106 as shown in FIG. 4.

In the following description, the assisted surgical planning system 100 in FIG. 1 is used as an example to describe its operation method.

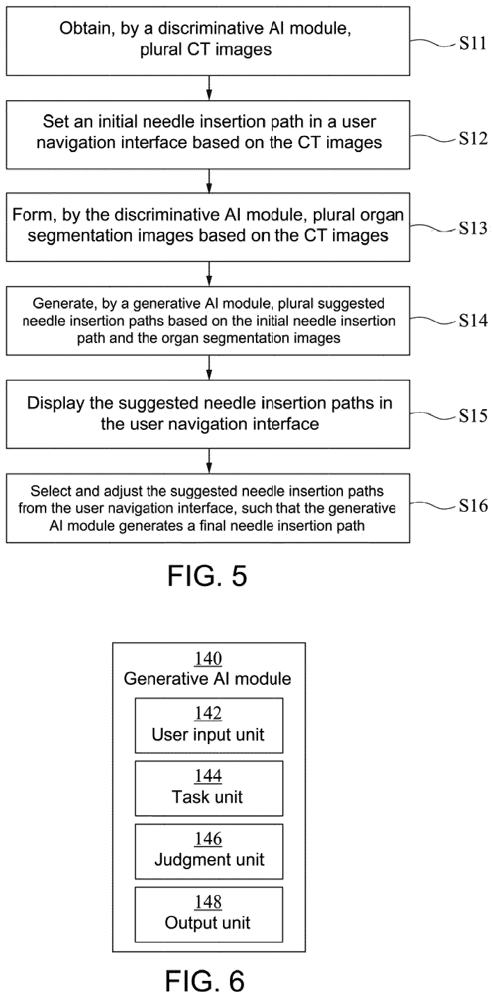

FIG. 5 illustrates a flow diagram of the operation method of the assisted surgical planning system according to one embodiment of the present disclosure. The operation method of the assisted surgical planning system includes the following steps. Referring also to FIGS. 1 and 5, in step S11, the discriminative AI module 120 obtains plural CT images (as shown in FIG. 3). Then, in step S12, an initial needle insertion path 102 is set in the user navigation interface 130 based on the CT image (see FIG. 3). Next, in step S13, the discriminative AI module 120 forms plural organ segmentation images based on the CT images. Then, in step S14, the generative AI module 140 generates plural suggested needle insertion paths 104 based on the initial needle insertion path 102 and the organ segmentation images (see FIG. 4). Subsequently, in step S15, the suggested needle insertion paths 104 are displayed in the user navigation interface 130. Finally, in step S16, the suggested needle insertion paths 104 are selected and adjusted from the user navigation interface 130, such that the generative AI module 140 generates a final needle insertion path 106 (see FIG. 4). In addition, the doctor can select and adjust the suggested needle insertion paths from the user navigation interface 130 to form a modified path. Then, the generative AI module 140 can generate plural other suggested needle insertion paths based on the modified path and the organ segmentation images for the doctor's reference.

Specifically, since the assisted surgical planning system 100 includes the generative AI module 140 and the host computer 110 connects the user navigation interface 130 to the generative AI module 140, the generative AI module 140 can generate the suggested needle insertion paths 104 (see FIG. 4) based on the initial needle insertion path 102 (see FIG. 3) and the organ segmentation images, and the user navigation interface 130 displays the suggested needle insertion paths 104 for the doctor's reference. The doctor can interact with the generative AI module 140 through the user navigation interface 130, and select and adjust the suggested needle insertion paths 104 from the user navigation interface 130, such that the generative AI module 140 generates the final needle insertion path 106 (see FIG. 4). In this way, the assisted surgical planning system 100 and the operation method thereof can effectively save the time of preoperative needle insertion path planning and reduce the influence of subjective opinions. In addition, the doctor can check, confirm, interact and adjust back and forth on customary two-dimensional (2D) images (e.g., organ segmentation images) when the assisted surgical planning system 100 performs needle insertion path planning, so that the correctness of the needle insertion paths is improved, and the surgical risk can be further reduced.

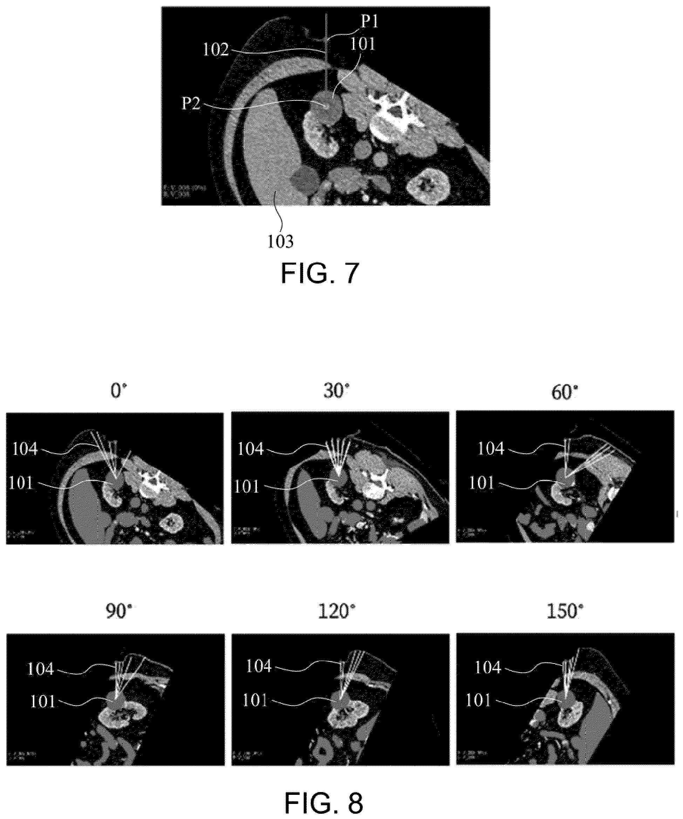

FIG. 6 illustrates a block diagram of the generative AI module 140 in FIG. 1. FIG. 7 illustrates an image of the generative AI module 140 in FIG. 6 when being contextually trained. Referring also to FIGS. 6 and 7, the generative AI module 140 includes a user input unit 142, a task unit 144, a judgment unit 146, and an output unit 148. In some embodiments, the generative AI module 140 can be contextually trained, that is, the generative AI module 140 is contextually learned and trained in a customized manner, such that the generative AI module 140 learns about the surgical context and its tasks, and provides a more clinically appropriate response, e.g., provides suggested needle insertion paths for the doctor to choose and adjust. Contextual training includes inputting an entry point P1 (e.g., a green point in FIG. 7), a target point P2(e.g., a yellow point in FIG. 7), plural danger zones 103 (e.g., pink and dark blue zones in FIG. 7), and a tumor 101(e.g., a red zone in FIG. 7) in a medical image (e.g., organ segmentation image) through the user input unit 142. In some embodiments, the danger zones 103 may include arteries, veins, and blood vessels.

FIG. 8 illustrates images of the generative AI module 140 when generating the suggested needle insertion paths 104 based on the initial needle insertion path 102 and the organ segmentation images according to one embodiment of the present disclosure. Then, the initial needle insertion path 102 (see FIG. 7) is adjusted through the task unit 144 to generate the suggested needle insertion paths 104. The task unit 144 is designed to perform fine adjustment of the paths, while the judgment unit 146 is designed to find a safer and more effective path (e.g., away from the danger zones), and can steer the suggested needle insertion paths 104 away from the danger zones 103 and adjust the target point P2 to the center of the tumor 101. In addition, there are more clinical considerations such as fat thickness in different zones, variables such as organ displacement caused by water pumping, etc. The contextual learning only enables the generative AI module 140 to learn the doctor's experience and then to perform better clinical path planning. Coordinates and images of the suggested needle insertion paths 104 can then be generated through the output unit 148. For example, the assisted surgical planning system 100 in FIG. 1 can capture the coordinates and display the coordinates in the user navigation interface 130 (see FIG. 1) for interaction with the doctor.

After the doctor has completed the planning of the initial needle insertion path 102 in FIG. 7, the assisted surgical planning system 100 (see FIG. 1) performs K-degree (e.g. 360-degree) rotation and reslicing along the path to provide more path possibilities in the space. In this way, n (e.g., six) resliced images of 0°, 30°, 60°, 90°, 120°, and 150° in FIG. 8 can be obtained after the rotation. The generative AI module 140 performs m path suggestions on the above n image slices, e.g., five suggested needle insertion paths 104 on each slice in FIG. 8. The doctor can then perform interaction and adjustment to the desired slices and paths.

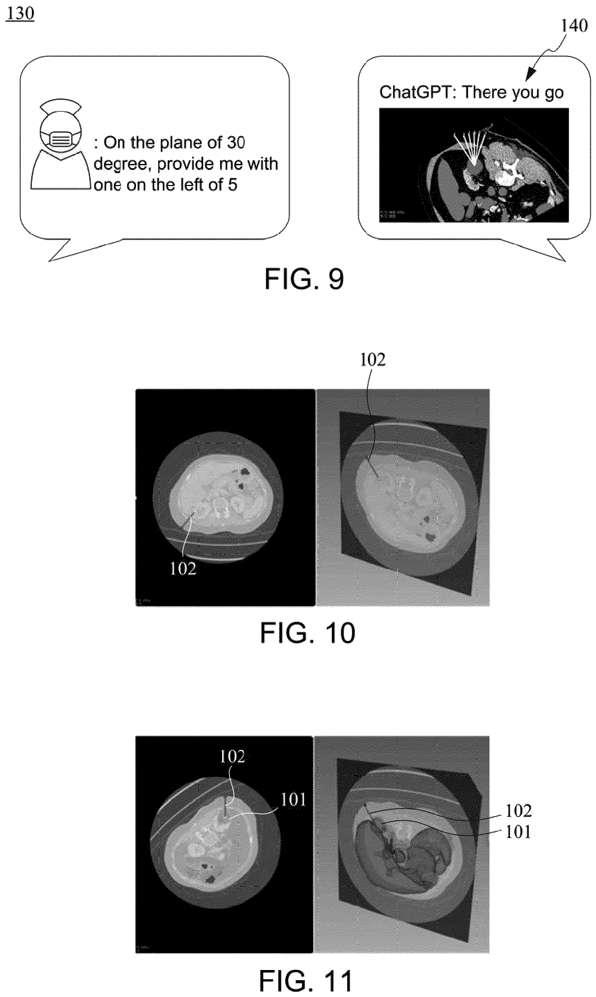

FIG. 9 illustrates a schematic diagram of interaction between the doctor and the generative AI module 140 (see FIG. 1) according to one embodiment of the present disclosure. A screen in FIG. 9 can be displayed in the user navigation interface 130 of FIG. 1. For example, the doctor considers one of a plurality of slices (e.g., 30° slice in FIG. 8) to be the most appropriate needle insertion slice, but in several suggestions provided by the generative AI module 140, a path 5 needs to be finely adjusted and asked to generate another path. After being instructed by the doctor's requirements, the generative AI module 140 can finely adjust the path 5 to generate a path 6 in response to the suggestion to meet the doctor's request.

FIG. 10 illustrates images when the initial needle insertion path 102 is set according to one embodiment of the present disclosure. FIG. 11 illustrates images when the organ segmentation images are formed according to one embodiment of the present disclosure. Referring also to FIGS. 10 and 11, the doctor can perform initial path planning in the CT images of FIG. 10, for example, providing the initial needle insertion path 102, and then the assisted surgical planning system 100 (see FIG. 1) can perform automated organ segmentation by the discriminative AI module 120 to obtain the organ segmentation images of FIG. 11.

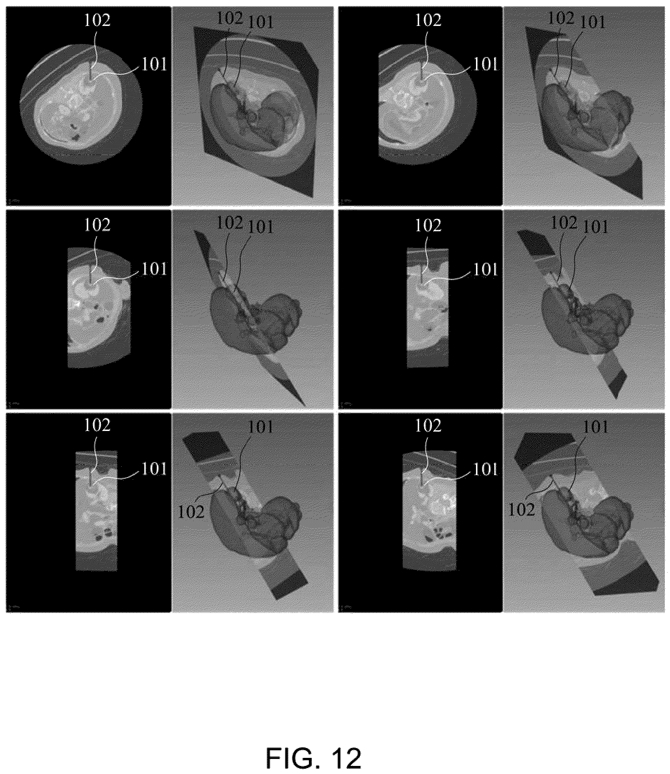

FIG. 12 illustrates images when K-degree image reslicing is performed along the initial needle insertion path 102 according to one embodiment of the present disclosure. Then, K-degree (e.g., 360 degree) image reslicing is performed along the initial needle insertion path 102 planned by the doctor.

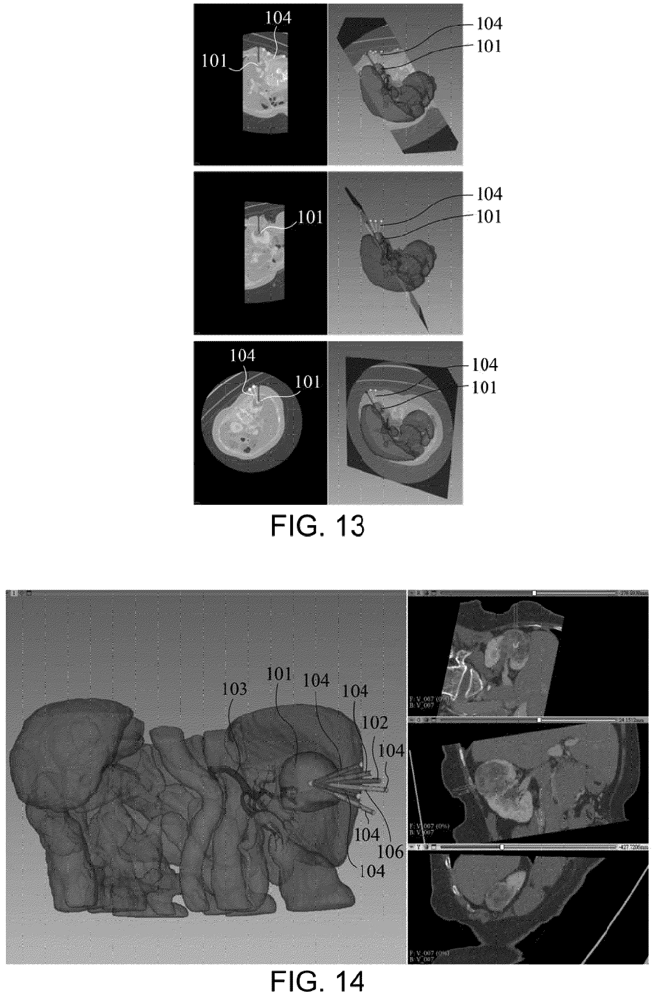

FIG. 13 illustrates images when the suggested needle insertion paths 104 are generated according to one embodiment of the present disclosure. Then, the generative AI module 140 (see FIG. 1) generates a plurality of suggested needle insertion paths 104 on each image cut.

FIG. 14 illustrates images when the final needle insertion path 106 is generated according to one embodiment of the present disclosure. Finally, the suggested needle insertion paths 104 generated by the generative AI module 140 (see FIG. 1) can be displayed in 3D (see the left half of FIG. 14) and 2D (see the right half of FIG. 14) images in the user navigation interface 130, so that the doctor can check and confirm back and forth on the customary CT images, and make interaction and adjustment. All information is visualized in 3D and 2D interfaces to facilitate the doctor to observe and finely adjust, where the 2D image provides a multi-planar reconstruction (MPR) view of the needle insertion paths in three directions. The 3D and 2D interfaces include the tumor 101 (e.g., red zones) and the danger zones 103 (e.g., pink and blue zones) automatically segmented. At the same time, the needle insertion paths are displayed, including the initial needle insertion path 102 (e.g., brown path) of the doctor, the suggested needle insertion paths 104 (e.g., blue paths) generated by the system, and the final needle insertion path 106 (e.g., bright green path) selected after interaction.

After the interaction between the doctor and the system to select the final needle insertion path 106 is completed, since the images read by the generative AI module 140 (see FIG. 1) are a plurality of two-dimensional image slices (i.e., CT images), the coordinates of the path when a path suggestion is made are also two-dimensional image coordinates. However, the system displays the location of a path in a 3D space in the user navigation interface 130 (see FIG. 1) and transmits the path to the robot 150 (see FIG. 1) for path tracking, so 2D path coordinates transmitted back by the generative AI module 140 can be converted to path coordinates in a 3D space. In some embodiments, when multi-angle reslicing of a two-dimensional image is performed, the system records the coordinates of the image in the space at each angle (i.e., $_{CT(n\ degree)}^{3D}T$ ), so that the path coordinates (i.e., $_{\square}^{CT(n\ degree)}P_{entry}$, $_{\square}^{CT(n\ degree)}P_{target}$ ) in any two-dimensional image (i.e.,) can be converted to path coordinates (i.e., $_{\square}^{3D}P_{entry}$, $_{\square}^{3D}P_{target}$ ) in the 3D space. A 2D-to-3D coordinate conversion formula is as follows:

$$^{3D}P_{entry} = {}_{CT}^{3D}T\ {}^{CT}P_{entry}$$

$$^{3D}P_{target} = {}_{CT}^{3D}T\ {}^{CT}P_{target}$$

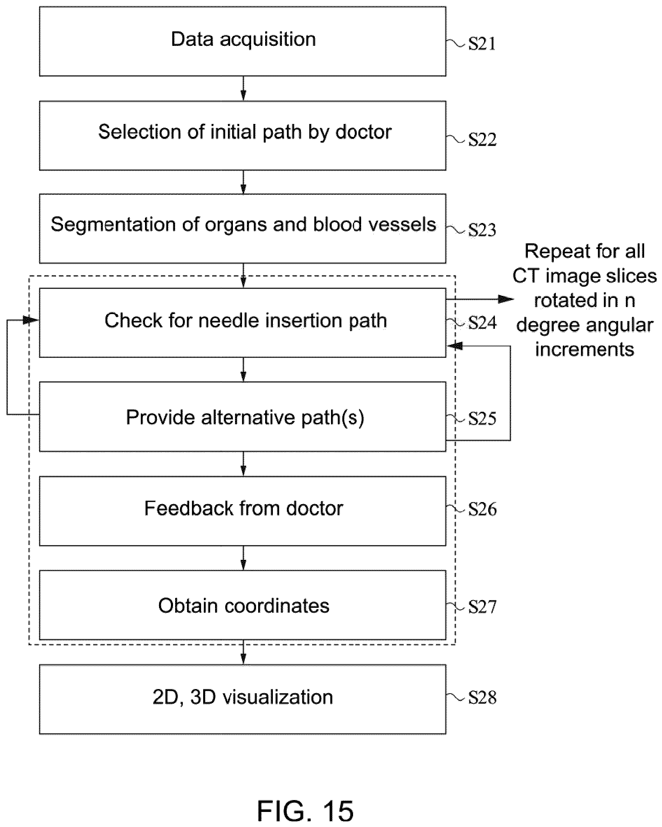

FIG. 15 illustrates a flow diagram of an operation method of an assisted surgical planning system according to another embodiment of the present disclosure. In step S21, data acquisition is performed, for example, CT images of a patient are obtained and displayed in an interface (e.g., the user navigation interface described above). Then, in step S22, a doctor sets an initial needle insertion path. Selection of an initial path by the doctor is performed on the CT images of the patient to provide an expected needle insertion zone. Then in step S23, segmentation of organs and blood vessels is performed. The assisted surgical planning system performs automatic organ segmentation to segment danger zones, tumors, etc., so that subsequently the generative AI module can identify the zones. In addition, the system then performs k-degree (e.g., 360-degree) image reslicing along the initial path. Then in step S24, check for the needle insertion path is performed. For example, a path safety check is performed to ensure the safety of the initial path and ensure that the initial path does not pass through the danger zones. Then, in step S25, alternative needle insertion paths (e.g., the suggested needle insertion paths described above) are provided. Then, in step S26, from feedback from doctor (e.g., adjustment and selection of the suggested needle insertion paths), fine adjustment of the needle insertion paths is performed through interaction with the doctor. Then, in step S27, coordinates (e.g., the coordinates of the final needle insertion path described above) are obtained. In addition, in steps S24-S27, a repeat for all CT image slices rotated in n degree angular increments can be performed. In step S28, 2D and 3D visualization (as displayed in the user navigation interface 130 described above) is performed. In other words, the coordinate position of the path finally selected after the interaction (the final needle insertion path described above) can be calculated by the system and displayed synchronously in the 2D and 3D interfaces (as shown in FIG. 14).

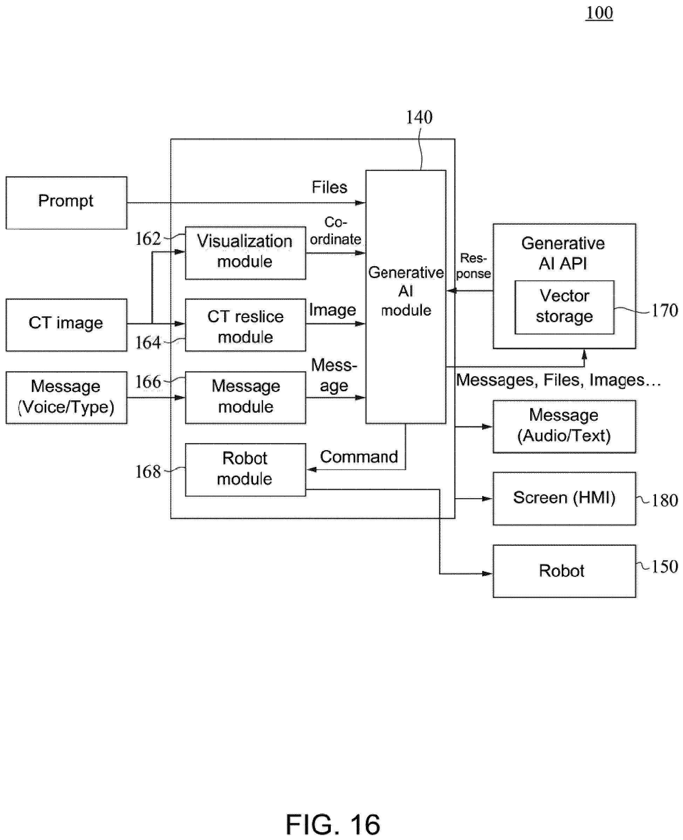

FIG. 16 illustrates a flow diagram of the assisted surgical planning system according to one embodiment of the present disclosure. The assisted surgical planning system 100 further includes a visualization module 162, a CT reslice module 164, a message module 166, a robot module 168, a robot 150, and a screen 180. The visualization module 162, the CT reslice module 164, the message module 166 and the robot module 168 are electrically connected to the generative AI module 140. The generative AI module 140 can receive prompts (e.g., files). The visualization module 162 can receive the CT images and the coordinates of the paths generated by the generative AI module 140, e.g., the coordinates of the suggested needle insertion paths and the coordinates of the final needle insertion path, and displays the paths on the screen 180. The CT reslice module 164 can receive the CT images and transmit the organ segmentation images to the generative AI module 140. The message module 166 can receive voice messages and type messages and transmits the messages to the generative AI module 140. The assisted surgical planning system 100 can transmit both audio messages and text messages. The robot module 168 can receive a command from the generative AI module 140 to operate the robot 150 having a puncture needle.

The generative AI module 140 can communicate with an application program interface (API) of a large language model (LLM), including sending images, commands and messages, and after receiving messages transmitted back, interpreting the messages, extracting the coordinates in the messages, and displaying the messages to the user, etc. The application program interface has a vector storage 170. In addition, whether an answer matches the user's question is checked. We write the application program interface into the back end of the system interface to interface with the generative AI module 140, so that the user can use the system and the back-end generative AI to make voice interaction, and the response and the generated paths of the generative AI can be automatically captured by the system and displayed in the system interface (e.g., the screen 180).

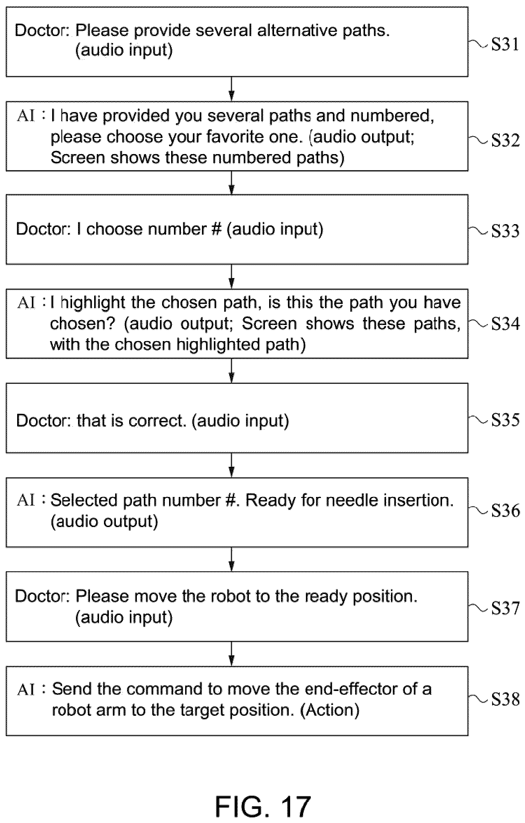

FIG. 17 illustrates a flow diagram of an operation method of an assisted surgical planning system according to yet another embodiment of the present disclosure. A process of interaction between a doctor and the assisted surgical planning system is as follows. In step S31, Doctor: Please provide several alternative paths. (audio input). Then in step S32, AI: I have provided you several paths and numbered, please choose your favorite one. (audio output; Screen shows these numbered paths). Then in step S33, Doctor: I choose number # (audio input). Then in step S34, AI: I highlight the chosen path, is this the path you have chosen? (audio output; Screen shows these paths, with the chosen highlighted path). Then in step S35, Doctor: that is correct. (audio input). Then in step S36, AI: Selected path number #. Ready for needle insertion. (audio output). Then in step S37, Doctor: Please move the robot to the ready position. (audio input). Finally, in step S38, AI: Send the command to move the end-effector of a robot arm to the target position. (Action).

**Claims**

1. An assisted surgical planning system, comprising:

   a host computer (110);
   a discriminative artificial intelligence (AI) module (120) electrically connected to the host computer (110), and configured to form a plurality of organ segmentation images based on a plurality of computed tomography (CT) images;
   a user navigation interface (130) electrically connected to the host computer (110), and configured to display the organ segmentation images, an initial needle insertion path, a plurality of suggested needle insertion paths, and a final needle insertion path; and
   a generative AI module (140) electrically connected to the host computer (110), such that the host computer (110) connects the user navigation interface (130) to the generative AI module (140), wherein the generative AI module (140) is configured to generate the suggested needle insertion paths based on the organ segmentation images and the initial needle insertion path, and display the suggested needle insertion paths in the user navigation interface (130).

2. The assisted surgical planning system according to claim 1, wherein the generative AI module (140) is configured for selection and adjustment in the user navigation interface (130) based on the suggested needle insertion paths to generate the final needle insertion path.

3. The assisted surgical planning system according to any one of claims 1-2, wherein the generative AI module (140) comprises a user input unit (142) configured to input an entry point, a target point, a plurality of danger zones and a tumor into the organ segmentation images.

4. The assisted surgical planning system according to claim 3, wherein the generative AI module (140) further comprises a task unit (144) configured to adjust the initial needle insertion path to generate the suggested needle insertion paths.

5. The assisted surgical planning system according to claim 4, wherein the generative AI module (140) further comprises a judgment unit (146) configured to steer the suggested needle insertion paths away from the danger zones and adjust the target point to a center of the tumor.

6. The assisted surgical planning system according to claim 5, wherein the generative AI module (140) further comprises an output unit (148) configured to generate coordinates and images of the suggested needle insertion paths.

7. The assisted surgical planning system according to any one of claims 1-6, further comprising:
   a visualization module (162) electrically connected to the generative AI module (140), and configured to receive the CT images, coordinates of the suggested needle insertion paths and coordinates of the final needle insertion path.

8. The assisted surgical planning system according to any one of claims 1-7, further comprising:
   a CT reslice module (164) electrically connected to the generative AI module (140), and configured to receive the CT images and transmit the organ segmentation images to the generative AI module (140).

9. The assisted surgical planning system according to any one of claims 1-8, further comprising:
   a message module (166) electrically connected to the generative AI module (140), and configured to transmit voice messages and type messages to the generative AI module (140).

10. The assisted surgical planning system according to any one of claims 1-9, further comprising:

a robot module (168) electrically connected to the generative AI module (140), and configured to receive a command from the generative AI module (140) to operate a robot having a puncture needle.

11. An operation method of an assisted surgical planning system, comprising:

obtaining, by a discriminative AI module (120), a plurality of CT images;
setting an initial needle insertion path in a user navigation interface (130) based on the CT images;
forming, by the discriminative AI module (120), a plurality of organ segmentation images based on the CT images;
generating, by a generative AI module (140), a plurality of suggested needle insertion paths based on the initial needle insertion path and the organ segmentation images, wherein the discriminative AI module (120), the generative AI module (140) and the user navigation interface (130) are electrically connected to a host computer (110);
displaying the suggested needle insertion paths in the user navigation interface (130); and
selecting and adjusting the suggested needle insertion paths from the user navigation interface (130), such that the generative AI module (140) generates a final needle insertion path.

12. The operation method of an assisted surgical planning system according to claim 11, further comprising:

selecting and adjusting the suggested needle insertion paths from the user navigation interface (130) to form a modified path; and
generating, by the generative AI module (140), a plurality of other suggested needle insertion paths based on the modified path and the organ segmentation images.

13. The operation method of an assisted surgical planning system according to any one of claims 11-12, further comprising:
contextually training the generative AI module (140).

14. The operation method of an assisted surgical planning system according to claim 13, wherein contextually training the generative AI module (140) comprises:
inputting an entry point, a target point, a plurality of danger zones and a tumor into the organ segmentation images, wherein the danger zones comprise arteries, veins, and blood vessels.

15. The operation method of an assisted surgical planning system according to claim 14, wherein contextually training the generative AI module (140) further comprises:

adjusting the initial needle insertion path to generate the suggested needle insertion paths; and
generating coordinates and images of the suggested needle insertion paths.

16. The operation method of an assisted surgical planning system according to claim 15, wherein adjusting the initial needle insertion path to generate the suggested needle insertion paths comprises:

steering the suggested needle insertion paths away from the danger zones; and
adjusting the target point to a center of the tumor.

17. The operation method of an assisted surgical planning system according to any one of claims 11-16, further comprising:
receiving, by a visualization module (162) electrically connected to the generative AI module (140), the CT images, coordinates of the suggested needle insertion paths and coordinates of the final needle insertion path.

18. The operation method of an assisted surgical planning system according to any one of claim 11-17, further comprising:
receiving the CT images and transmitting the organ segmentation images to the generative AI module (140) by a CT reslice module (164) electrically connected to the generative AI module (140).

19. The operation method of an assisted surgical planning system according to any one of claims 11-18, further comprising:
transmitting, by a message module (166) electrically connected to the generative AI module (140), voice messages and type messages to the generative AI module (140).

**20.** The operation method of an assisted surgical planning system according to any one of claims 11-19, further comprising:
receiving, by a robot module (168) electrically connected to the generative AI module (140), a command from the generative AI module (140) to operate a robot having a puncture needle.

200

100

130

140

User navigation interface

Generative AI module

Discriminative AI module

Host computer

Robot

120

110

150

# FIG. 1

S1

S2

S3

S5

The doctor performs initial path planning

The discriminative AI performs organ segmentation

The generative AI performs a path suggestion

Final path

The doctor selects and adjusts

S4

# FIG. 2

FIG. 3

FIG. 4

Obtain, by a discriminative AI module,
plural CT images — S11

Set an initial needle insertion path in a user
navigation interface based on the CT images — S12

Form, by the discriminative AI module, plural organ
segmentation images based on the CT images — S13

Generate, by a generative AI module, plural suggested
needle insertion paths based on the initial needle insertion
path and the organ segmentation images — S14

Display the suggested needle insertion paths in
the user navigation interface — S15

Select and adjust the suggested needle insertion paths
from the user navigation interface, such that the generative
AI module generates a final needle insertion path — S16

FIG. 5

140
Generative AI module

142
User input unit

144
Task unit

146
Judgment unit

148
Output unit

FIG. 6

FIG. 7

FIG. 8

FIG. 9

FIG. 10

FIG. 11

FIG. 12

**FIG. 13**

**FIG. 14**

Data acquisition ∿ S21

Selection of initial path by doctor ∿ S22

Segmentation of organs and blood vessels ∿ S23

Check for needle insertion path ∿ S24

Repeat for all CT image slices rotated in n degree angular increments

Provide alternative path(s) ∿ S25

Feedback from doctor ∿ S26

Obtain coordinates ∿ S27

2D, 3D visualization ∿ S28

FIG. 15

100

FIG. 16

Doctor: Please provide several alternative paths. (audio input) ～S31

AI : I have provided you several paths and numbered, please choose your favorite one. (audio output; Screen shows these numbered paths) ～S32

Doctor: I choose number # (audio input) ～S33

AI : I highlight the chosen path, is this the path you have chosen? (audio output; Screen shows these paths, with the chosen highlighted path) ～S34

Doctor: that is correct. (audio input) ～S35

AI : Selected path number #. Ready for needle insertion. (audio output) ～S36

Doctor: Please move the robot to the ready position. (audio input) ～S37

AI : Send the command to move the end-effector of a robot arm to the target position. (Action) ～S38

FIG. 17

| | Europäisches Patentamt / European Patent Office / Office européen des brevets | **EUROPEAN SEARCH REPORT** | **Application Number** EP 25 19 0141 |
|---|---|---|---|

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2023/165638 A1 (MANSI TOMMASO [US] ET AL) 1 June 2023 (2023-06-01) * paragraphs [0005], [0022], [0027] - [0031], [0060], [0101] - [0106]; figures 1-6,10 * ----- | 1-20 | INV. A61B34/10 G06N3/08 G06T7/10 ADD. A61B34/30 |
| A | US 2023/008386 A1 (NAHUM BERTIN [FR] ET AL) 12 January 2023 (2023-01-12) * paragraphs [0102] - [0104], [0121]; figure 2 * ----- | 1-20 | |
| A | US 2022/133484 A1 (LANG PHILIPP K [US]) 5 May 2022 (2022-05-05) * paragraphs [0082] - [0086], [0157] * ----- | 1-20 | |

TECHNICAL FIELDS SEARCHED (IPC)

G06N
G16H

The present search report has been drawn up for all claims

| Place of search | Date of completion of the search | Examiner |
|---|---|---|
| Munich | 4 November 2025 | Maier, Christian |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...........................................................................
& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 19 0141

04-11-2025

| Patent document cited in search report | Publication date | Patent family member(s) | | Publication date |
|---|---|---|---|---|
| US 2023165638 A1 | 01-06-2023 | CN | 116172699 A | 30-05-2023 |
| | | EP | 4186455 A1 | 31-05-2023 |
| | | US | 2023165638 A1 | 01-06-2023 |
| US 2023008386 A1 | 12-01-2023 | CA | 3153174 A1 | 24-06-2021 |
| | | CN | 113966204 A | 21-01-2022 |
| | | EP | 4078464 A1 | 26-10-2022 |
| | | ES | 2972216 T3 | 11-06-2024 |
| | | FR | 3104934 A1 | 25-06-2021 |
| | | IL | 293534 A | 01-08-2022 |
| | | JP | 2023506353 A | 16-02-2023 |
| | | JP | 2025060909 A | 10-04-2025 |
| | | KR | 20220117209 A | 23-08-2022 |
| | | US | 2023008386 A1 | 12-01-2023 |
| | | WO | 2021123651 A1 | 24-06-2021 |
| US 2022133484 A1 | 05-05-2022 | US | 11278413 B1 | 22-03-2022 |
| | | US | 11660197 B1 | 30-05-2023 |
| | | US | 11938030 B1 | 26-03-2024 |
| | | US | 2022133484 A1 | 05-05-2022 |

EPO FORM P0459